Europäisches Patentamt

European Patent Office

Office européen des brevets

(11) Publication number: **0 302 673 B1**

# EUROPEAN PATENT SPECIFICATION

(45) Date of publication of patent specification: **07.10.92**  (51) Int. Cl.⁵: **G01N 33/53**, G01N 33/541, G01N 33/532, //G01N33/569

(21) Application number: **88307028.6**

(22) Date of filing: **29.07.88**

The file contains technical information submitted after the application was filed and not included in this specification

(54) **System and process for a visible assay for analyte.**

(30) Priority: **06.08.87 US 82106**

(43) Date of publication of application:
**08.02.89 Bulletin  89/06**

(45) Publication of the grant of the patent:
**07.10.92 Bulletin  92/41**

(84) Designated Contracting States:
**AT BE CH DE ES FR GB GR IT LI NL SE**

(56) References cited:
**EP-A- 0 154 749**
**EP-A- 0 196 880**
**FR-A- 2 523 311**
**US-A- 4 582 810**

**CHEMICAL ABSTRACTS, vol. 96, no. 7, February 15, 1982, Columbus, OH (US); M.A.APICELLA et al, p. 443, no. 50284y**

**MEDLINE, Telesystems Ouestel; no. 88199530**

**MEDLINE, Telesystems Ouestel; no. 80182607**

(73) Proprietor: **Becton Dickinson and Company**
**One Becton Drive**
**Franklin Lakes New Jersey 07417-1880(US)**

(72) Inventor: **Lovell, Stephen James**
**724A7 Camberley Circle**
**Towson, MD(US)**
Inventor: **Bruton, Jeffery Hayden**
**3929 Lumo Circle**
**Randallstown, MD(US)**

(74) Representative: **Ruffles, Graham Keith et al**
**MARKS & CLERK 57-60 Lincoln's Inn Fields**
**London WC2A 3LS(GB)**

## Description

This invention relates to an assay for a ligand to be determined (analyte), and to products used in such assay. More particularly, the present invention relates to a solid phase assay.

In a solid phase assay, it is known to determine an analyte by a so called sandwich assay technique. In such a sandwich assay, a binder for the ligand to be determined (analyte) is supported on a solid support, and in the assay, there is formed a complex of such supported binder, analyte and tracer, which tracer is comprised of a binder to the analyte labelled with a detectable label.

French Patent 2,523,311, assigned to Institute Pasteur describes an indirect sandwich assay wherein there is produced in the assay a complex of binder, analyte, coupled compound comprised of a binder for the analyte and albumin, and a tracer comprised of labelled anti-albumin. Labels exemplified in this patent include enzymes, fluorochromes, radioactive elements, red blood cells and lectins.

EP-A-154 749 describes a process for assaying an analyte wherein a solid support supports a binder for an analyte and a tracer, for competitive binding with the analyte, comprises a visible particulate label. Results are detectable visually in this competitive binding assay.

US 4,582,810 describes a suspension of diagnostic particles which comprise antibody molecules attached to a carboxylate derivatised polymer core. Antibodies are linked to the core via an avidin-biotin bridge. The particles are useful in agglutination test.

The present invention is directed to providing an improved indirect sandwich assay for determining analyte which utilizes a tracer including a particulate label.

In accordance with the invention, there is provided an assay in which analyte is determined (qualitatively and/or quantitatively) by an indirect sandwich assay technique wherein in the assay there is formed a complex of (i) a binder for the analyte supported on a solid support; (ii) analyte; (iii) a coupled compound comprised of a binder specific for the analyte and a ligand; and (iv) a tracer comprised of a binder for the ligand of the coupled compound labelled with a visible particulate label which is a liposome containing a detectable marker. At least the portion of the solid support on which the binder is supported has a surface area (area to unit weight of material) such that the binder can be supported on the test area in a concentration (weight to unit area) such that the tracer is visible under the assay conditions. The term "visible" as used herein means that the label can be seen without the use of instrumentation; i.e., with the naked eye. Such assay will be referred to herein as a "visible indirect sandwich assay" or "visible assay".

The test area which is employed in the visible assay is generally formed from a cellulose ester with nitrocellulose giving exceptionally good results. It is to be understood that the term "nitrocellulose" refers to nitric acid esters of cellulose, which may be nitrocellulose alone, or a mixed ester of nitric acid and other acids, and in particular, aliphatic carboxylic acids having from one to seven carbon atoms, with acetic acid being preferred. Such solid supports which are formed from cellulose esterified with nitric acid alone, or a mixture of nitric acid and another acid such as acetic acid, are often referred to as nitrocellulose paper.

Although nitrocellulose is a preferred material for the test area, it is to be understood that other materials, having a surface area sufficient for supporting the binder in a concentration as hereinabove described may also be employed for such test areas.

In general, the test area which is used in the visible assay has a surface area such that it is capable of supporting binder in a concentration of at least 1ug/cm$^2$, (most generally in a concentration of at least 10ug/cm$^2$) and preferably at least 40ug/cm$^2$.

Thus, in accordance with an aspect of the present invention, there is provided a sandwich assay in which a sandwich is formed comprised of: support-binder-analyte-coupled compound-tracer, wherein the coupled compound is formed from two ligands, with one of the ligands being a binder for the analyte and the other ligand is bound by the tracer.

The coupled compound is comprised of a binder for the analyte, which is generally an antibody against the analyte (polyclonal and/or monoclonal) and a ligand for which there is a binder.

The ligand of the coupled compound may be any one of a variety of ligands for which a binder exists. Thus, for example, the ligand of the coupled compound may be biotin, dinitrophenol, trinitrophenol, fluorescein isothiocyanate, fluorescein isocyanate, peroxidase, alkaline phosphatase, albumin, ferritin etc. The ligand portion of the coupled compound is generally a hapten.

The coupled compound may be produced by conjugating a binder for the analyte with an appropriate ligand by procedures known in the art, e.g., by use of an appropriate coupling or spacer compound having two reactive functional groups, one of which is capable of being linked to a functional group of the binder and the second of which is capable of being linked to a functional group of the appropriate ligand. In some cases, it may be possible to directly couple or conjugate the binder with the ligand to provide a coupled compound; e.g., by coupling a reactive functional group of the binder with a reactive functional group of the appropriate ligand.

The tracer is comprised of a binder for the ligand of the coupled compound and is preferably an antibody (monoclonal or polyclonal). Thus, in the assay, the tracer will be bound by the coupled compound; the coupled compound will be bound by the analyte; and the analyte will be bound by the supported binder on the solid support.

In accordance with a further aspect of the present invention, there is provided a reagent kit which includes a test substrate, as hereinabove described, having a binder supported in the test area of the substrate; a coupled compound, as hereinabove described; and a tracer, as hereinabove described, comprised of a binder for the ligand of the coupled compound labeled with a visible particulate label. The reagent kit may also include standards (samples having known concentrations of analyte), buffers, wash solutions and the like. The reagents may be included in suitable containers, such as test vials.

As hereinabove indicated, in producing the tracer for the visible assay the binder for the ligand is labeled with a particulate label, which is a liposome containing a detectable marker.

As known in the art, liposomes can be prepared from a wide variety of lipids, including phospholipids, glycolipids, steroids, relatively long chain alkyl esters; e.g.,. alkyl phosphates, fatty acid esters, lecithin, fatty amines and the like. A mixture of fatty materials may be employed, such as a combination of neutral steroid, a charged amphiphile and a phospholipid. As illustrative examples of phospholipids, there may be mentioned lecithin, sphingomyelin, dipalmitoyl lecithin, and the like. As representative steroids, there may be mentioned cholesterol, cholestanol, lanesterol, and the like. As representative examples of charged amphiphilic compounds, which generally contain from 12 to 30 carbon atoms, there may be mentioned mono- or dialkyl phosphate ester or an alkylamine; e.g., dicetyl phosphate, stearyl amine, hexadecyl amine, dilauryl phosphate, and the like.

The liposome sacs are prepared in an aqueous solution including the marker whereby the sacs will include the marker in the interior thereof. The liposome sacs may be prepared by vigorous agitation in the solution, followed by removal of marker from the exterior of the sac.

Further details with respect to the preparation of liposomes are set forth in U.S. Patent No. 4,342,826 and PCT International Publication No. WO80/01515, both of which are hereby incorporated by reference.

As hereinabove indicated, the marker included in the sac may be a dye or some other material which is visible, without lysing of the sacs.

The binder for the ligand of the coupled compound may be labeled with the particulate label so as to produce a tracer for use in the invention by procedures generally known in the art, with the procedure which is used being dependent upon the binder and the particulate label which is employed. Such techniques include, covalent coupling, derivatization or activation, and the like. In producing a tracer wherein the binder is labeled with a sac, the sac may be produced from a component which has been derivatized with a binder, whereby the sac, when produced, is sensitized with the binder. In another procedure, the sac including the marker may be initially formed, followed by sensitizing the sac with binder by procedures known in the art.

Thus, the tracer is comprised of a binder for the ligand of the coupled compound and a particulate label, and the particulate label provides a tracer which is visible under the assay conditions so that the presence and/or amount of analyte may be determined without further treatment and without the use of instrumentation; e.g., by use of a liposome containing a dye as the particulate label.

In accordance with a preferred aspect of the present invention, the binder for the analyte which is supported on the test area of the test substrate is preferably in a defined area of such test area such as, for example, in the form of a spot, rather than being dispersed over the entire test area of the test substrate. As hereinabove indicated, in accordance with a preferred embodiment, the binder for the analyte supported on the test area is preferably present in at least one microgram per $cm^2$, most generally at least 10 micrograms per $cm^2$, and preferably 40 micrograms per $cm^2$. The residual binding capacity of the test area of the overall test substrate may be saturated or blocked by treatment of the test area with one or more types of proteins which do not specifically bind materials to be employed in the assay. Thus, for example, residual binding capacity may be blocked by use of bovine serum albumin.

In some cases, in applying the binder for the analyte (in particular an antibody) to the test area, a polyhydroxy compound (glycerol, erythritol, sorbitol, etc.) or a sugar (glucose, sucrose, etc.) is included in the antibody solution to prevent non-specific binding (false positives) during the assay.

In accordance with a preferred embodiment, the test substrate includes a test layer having the test area for supporting the binder for the analyte in a concentration whereby the tracer used in the assay, when bound to the test area, under assay conditions is visible in the test area, without further treatment and also includes a flow controlling layer, beneath the test layer, which is formed of a porous material having a pore size to control the rate of flow of assay reagents through the test substrate, when such reagents are applied to the first layer. The test substrate also preferably includes a porous spacer layer for spacing an absorbent layer, formed of an absorbent material, from the flow controlling layer.

The absorbent layer has an absorbency sufficient to absorb the reagent liquids applied to the test layer during the assay. In addition, the absorbent material functions to provide for flow through the test substrate.

It is understood that the entire test layer may be formed of the test area material or, alternatively, only the test area of the test layer may be formed of such a material.

In addition, since the test substrate is employed in a manner such that the assay reagents flow through the layers of the test substrate, the test area has a pore size which is greater than the size of the particulate label employed in the assay so that portions of the tracer, which do not become bound under assay conditions, flow into the test substrate and are not visible in the test area. In general, the test area should have a pore size which is at least 3 um, and most preferably at least 5 um. In general, the pore size does not exceed 12 um. It is to be understood, however, that although the previously described pore sizes are preferred, other pore sizes may be employed, depending upon the materials used in the assay.

The flow control layer of the test substrate is formed of a porous material which is employed to control the rate of flow of assay reagents through the test area and into the test substrate. The porous material which is employed in forming the flow control layer has a pore size which is less than the pore size of the material employed for forming the test area. Thus, in effect, the flow control layer functions to reduce the rate of flow of assay reagents through the more porous test area.

In general, the flow control layer, which functions to control the rate of flow of assay reagents through the test area and into the test substrate has a pore size of at least 0.5 micron, and in general does not have a pore size in excess of 10 microns.

The pore size of the flow control layer, as well as the thickness of the flow control layer are preferably controlled in a manner such that the flow of assay reagents through the test area provides the requisite sensitivity as well as a rapid and accurate assay.

The pore size and corresponding rate of flow selected for the second layer is dependent upon the expected range of analyte concentration. As the expected range of analyte concentration increases, the pore size and flow rate may increase. In fact, for some analytes which may be present in high concentrations, the second layer of the substrate may be omitted.

In accordance with one embodiment, the layer for controlling rate of flow through the test substrate is dimensioned and sized in a manner such that the flow rate of materials through the test area is in the order of at least 0.5 ml/min, and generally no more than 2 ml/min. It is to be understood, however, that the scope of the present invention is not limited to such flow rates.

The flow control layer is preferably formed from a non-fibrous material and preferably has pores or channels that provide for unidirectional flow from the test layer to the layer beneath the flow control layer; i.e., the flow control layer has defined flow channels which direct flow through the layer and minimize flow across the layer. The pores or channels preferably are of uniform size. The flow control layer is preferably formed from a polycarbonate.

Immediately below the flow control layer of the test substrate, there is provided a spacer layer of porous material which functions as a spacer between the flow controlling layer, and a porous absorbent layer. The spacer layer primarily functions to prevent materials which have passed through the top layers of the test substrate and into the absorbent layer of the test substrate from backing up into the top layers. To this end, the porous layer, which functions as a spacer, has a thickness which is sufficient to prevent materials which have passed into the absorbent layer from flowing back up into the top layers under assay conditions. In addition, the porous spacer layer has a pore size greater than the pore size of the flow controlling layer so that the spacer layer does not function to restrict flow through the test substrate.

It is to be understood, however, that in some cases, it may be possible to eliminate the spacer layer, provided that the characteristics of the remaining layers of the substrate; i.e., the test area, flow controlling layer and absorbent layer are such that the risk of material backing up into the test area from the absorbent layer is essentially eliminated. It is to be understood, however, that the use of a spacer layer is preferred. In the case where the flow control layer is not employed, the spacer layer spaces the test area from the absorbent layer.

The test substrate also includes an absorbent layer (fourth layer), which is a porous material which has an absorbing or absorbent capacity sufficient to absorb the liquid test reagents or materials which flow into the test substrate during the assay. The absorbent layer also functions to provide a driving force (concentration differential) which causes reagents applied to the test area to flow into the substrate; i.e., into the absorbent layer.

Thus, in accordance with the present invention, there is provided an assay which employs a tracer wherein the label portion of the tracer is a visible particulate label, and wherein the assay is performed on a test substrate, which is preferably formed from a plurality of layers of material having different characteristics, as hereinabove described, and wherein the assay reagents flow through the test area of the test substrate.

The materials which are employed in forming the various layers of the substrate are selected to have the hereinabove described characteristics. In addition, such materials should not produce non-specific binding of analyte or tracer. The materials may inherently have such characteristics, or alternatively, the materials may be treated to prevent nonspecific binding; for example, treatment with an appropriate protein, such as bovine serum albumin. The test area of the substrate is preferably also treated with a wetting agent in order to insure proper flow of the assay reagents through the test area and into the test substrate. As representative examples of wetting agents, there may be mentioned: sucrose, glycerol, glucose, sorbitol, etc. The test area may be simultaneously treated with a protein and wetting agent; e.g., an aqueous solution of BSA and sucrose.

In general, the test substrate is supported on or in a suitable support, such as a card, or a container. The selection of a suitable support for the test substrate is deemed to be within the scope of those skilled in the art from the teachings herein.

In addition, the test substrate is generally provided with a covering material, which directs assay reagents to the test area in which the binder is supported. Thus, for example, the substrate may be covered with a card including an aperture which overlies the portion of the test area which includes the binder whereby the various assay reagents are applied directly to the portion of the test area which includes the binder. Alternatively, the test substrate may be placed in a container, including a suitable aperture for directing assay reagents to the test area of the substrate.

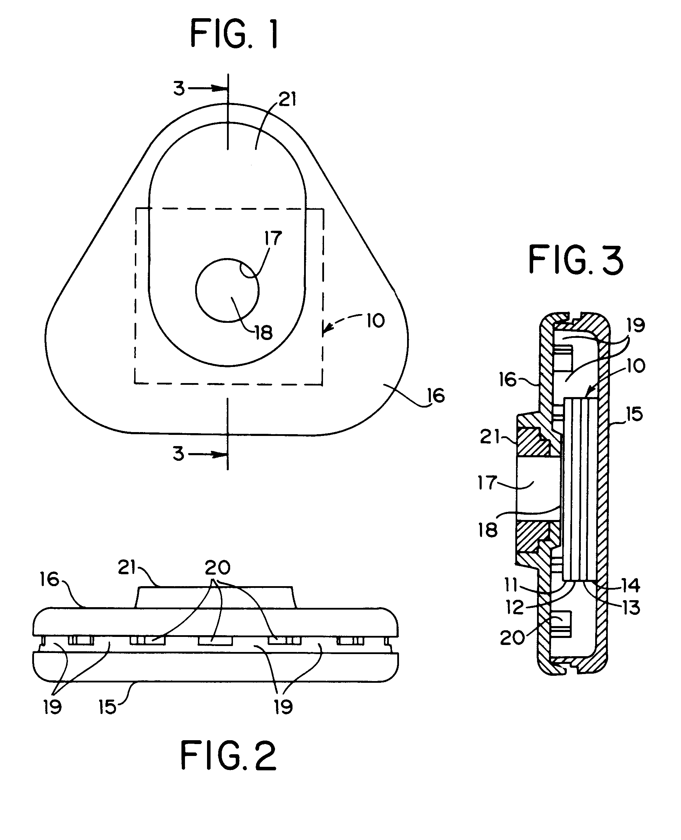

The present invention will be further described with respect to an embodiment thereof illustrated in the accompanying drawing, wherein:

The drawing is a simplified schematic diagram of a preferred embodiment of the present invention.

It is to be understood, however, that the scope of the invention is not to be limited thereby.

Referring now to the drawing, there is shown a test substrate, generally designated as 10, which is comprised of a first layer, generally designated as 11, which has a test area for supporting a binder in a concentration whereby the tracer in which the label is a particulate visible label, when bound, is visible under assay conditions. A preferred material is nitrocellulose, which has a pore size in excess of 2 microns , and generally less than 12 microns.

Immediately underneath the layer 11 and in contact therewith, there is a second layer 12, which functions to control flow of materials through the test substrate. The second layer 12 has a pore size less than the pore size of the layer 11, and has characteristics as hereinabove described. The layer is preferably formed from a polycarbonate.

Immediately underneath layer 12 and in contact therewith there is provided a layer 13, which functions as a spacer layer, as hereinabove described.

The layer 13 is formed of a porous material, and generally has a pore size greater than the pore size of layer 12. The layer 13 may be formed, for example, from a non-woven polyacetate. Immediately underneath layer 13 and in contact therewith, there is provided a layer 14, which is formed from an absorbent material, as hereinabove described. The layer 14 is preferably formed from a cellulose material, e.g., absorbent cellulose paper.

Thus, the test substrate is comprised of layers 11, 12, 13 and 14, which are combined to produce a unified substrate 10. The layers may be attached to each other, for example, by sewing of the layers to each other; however, other methods of attachment are possible.

As particularly shown, the test substrate 10 is in a test container which includes a base portion 15, and a cover portion 16. The base portion 15 has a depth such that the substrate 10 is within the container. In the preferred embodiment the container has three sides in a generally triangular shape with rounded corners.

The cover 16 which overlies layer 11, substrate 10, and test area 18 includes raised portion having a suitable aperture 17 which overlies the portion of the layer 11, which includes the supported binder, with such portion of the test area, schematically generally being indicated as 18.

The cover 16 is supported over layer 11 by toothlike projections 19 extending upward from the sides of the base portion 15. The projections 19 are of sufficient height so as to provide air spaces 20 which provide for ventilation of the sides of the substrate 10. The air spaces 20 are bounded by the projections 19, the cover 16, and the base portion 15.

The raised portion of the cover 16 surrounding the aperture 17 includes a colored area 21, the color of which contrasts from that of the cover 16 and the color to be generated in test area 18 to provide for a better reading of the test results which are generally determined by color. In the preferred embodiment, base portion 15, cover 16, and colored area 21 are made of plastic materials.

Thus, as shown, the binder supported on the layer 11 occupies only a portion of the entire layer 11, and the cover 16, including aperture 17 functions to direct the materials used in the assay into contact with the supported binder 18, with such materials then flowing through the test substrate to the absorbent layer 14 by sequentially flowing through layers 11, 12 and 13.

For example, in using the test substrate 10 for a sandwich assay, the supported binder may be an antibody specific for the analyte to be determined. If the sandwich assay is to be operated in a sequential mode, initially, sample which contains or is suspected of containing the analyte is applied to the substrate through the aperture 17 in cover 16, whereby the sample contacts the binder in area 18, with the sample flowing through the substrate to the absorbent layer 14. The analyte present in the sample will become specifically bound to the binder in area 18.

A coupled compound comprised of a binder for the analyte and a ligand which is bound by the binder of the tracer used in the assay is applied to the test substrate and becomes bound to bound analyte.

Thereafter, tracer is applied to the test substrate through the aperture 17 in cover 16, with such tracer being comprised of a binder, which binds to the ligand of the coupled compound and which is labeled with a visible particulate label. The tracer becomes bound to the coupled compound and any unbound portion flows through the test substrate to the absorbent layer 14.

If desired, a wash solution may be applied to the test substrate 10 prior to addition of the tracer.

Similarly, after addition of the tracer, a wash solution may be applied to the test substrate to wash any tracer which may not be specifically bound to the complex in area 18, into the absorbent layer 14.

The aperture 17, if desired, may be provided with an insert, which includes a filter for filtering assay materials to be applied to the test area.

The presence of color in area 18 is indicative of the presence of analyte, and if the assay is to be a quantitative assay, the quantity of such color is indicative of the quantity of analyte present in the sample.

Although in a preferred embodiment, the assay is accomplished in "flow through" format in which the binder is supported on an appropriate substrate and sample, coupled compound and tracer are caused to flow through the substrate, it is possible to employ other assay formats, e.g., contacting the supported binder without causing the reagents to flow through the substrate, such as use of a text card, or a chromatographic or capillary flow over the surface of the test substrate, etc.

Similarly, although it is preferred to employ a test substrate of the configuration hereinabove described when using a flow through assay, it is possible to use other configurations; e.g., a combination of test substrate and absorbent material without the flow control and spacer layers.

The indirect sandwich assay may be accomplished in a variety of formats.

For example, the analyte and coupled compound may be contacted with each other and the resulting mixture contacted with the supported binder. As a further alternative, it may be possible to contact tracer, coupled compound and sample, and the resulting mixture contacted with the supported binder.

The present invention will be further described with respect to the following examples; however, the scope of the invention is not to be limited thereby:

EXAMPLE I

A. Biotinylation of (lysine) amino groups of antibody.

This procedure uses N-hydroxysuccinimidobiotin (NHS-biotin) which is available commercially (e.g., Sigma Chemical Company). The biotinylation procedure is as follows:

(i) NHS-biotin (4 mg/ml in dimethyl sulfoxide) is added dropwise, with stirring to antibody solution (1 mg/ml in 0.1 M $NaHCO_3$, pH 8.2) in the ratio 1:20 (v/v). The mixture is allowed to stand for 4 hours at room temperature.

(ii) After 4 hours, 0.1 volume of 1 M glycine ethyl ester (pH 7.5) is added and the solution dialyzed exhaustively against 0.1 M $NaHCO_3$ to remove unattached biotin.

B. Biotinylation of carbohydrate fraction of antibody.

(i) Antibody is dialyzed against 10 mM Na phosphate buffer pH 7.4 containing 0.15 M NaCl. After dialysis the concentration of antibody is adjusted to 1 mg/ml and the pH is adjusted to 5.5 by the addition of 1 M acetate buffer pH 4.5.

(ii) Sodium periodate (100 mM in 20 mM sodium acetate/0.15 M NaCl pH 5.5) is added to the solution of antibody to a final concentration of 10 mM. Oxidation is allowed to continue for 15-30 min at room temperature, after which time the reaction is quenched by the addition of ethylene glycol (to 0.1 M).

(iii) The antibody solution is passed down a G-25 column equilibrated with 20 mM acetate/0.15 M NaCl pH 5.5. Fractions containing antibody are pooled.

(iv) Biotin hydrazide (approximately 5 mg/ml in DMSO) is added to the pooled antibody such that the final concentration of biotin hydrazide is 0.25 mg/ml. The antibody concentration at this step is 0.3-0.5 mg/ml. The mixture is allowed to stand for 24 hours at 4°C and is then dialyzed against 0.1 M NaHCO₃ pH 8.3 to remove unbound biotin hydrazide.

EXAMPLE II

An assay system for N. gonorrhoeae is as follows:

step (i) Monoclonal antibody against N. gonorrhoeae (MAb 148.1) is spotted on 5 um nitrocellulose - 3 ul spotted at 500 ug/ml. The membrane is then allowed to dry for 15 min at room temperature.

step (ii) The spotted membrane is then blocked by incubation in PBS containing 0.5% (w/v) low fat dried milk for 60 min at 37°C.

step (iii) The blocked membrane is washed with PBS to remove excess blocking solution and allowed to dry at room temperature.

step (iv) The membrane is then placed on absorbent paper.

step (v) N. gonorrhoeae antigen is then passed through the membrane. A fraction of the antigen is captured by the spotted MAb 148.1. Up to 1 ml of antigen in 1% BSA/1% octyl beta-D-glucopyranoside/30% goat serum/0.2% dried milk/PBS (pH 7.4) can be passed through the membrane.

step (vi) Biotinylated rabbit anti-N. gonorrhoeae antibody prepared by a procedure of Example I (100 ul of 10 ug/ml) is then passed through the membrane. This antibody recognizes antigen captured by the MAb 148.1. The biotinylated rabbit antibody is diluted with the same buffer used in step (v).

step (vii) A solution containing liposome coated with goat anti-biotin antibody is then passed through the membrane (100 ul of liposomes are used). These liposomes, which contain sulforhodamine, bind to the biotinylated antibody and provide the signal (red color).

step (viii) PBS (200 ul) is passed through the membrane to wash out unattached liposomes. The color intensity increases with increasing concentration of antigen.

The present invention is applicable to procedures and products for determining a wide variety of analytes. As representative examples of types of analytes, there may be mentioned: drugs, including therapeutic drugs and drugs of abuse; hormones, vitamins, proteins, including antibodies of all classes; peptides; steroids; bacteria; fungi; viruses; parasites; components or products of bacteria, fungi, viruses, or parasites; allergens of all types; products or components of normal or malignant cells; etc. As particular examples, there may be mentioned T₄; T₃; digoxin; hcG; insulin; theophylline; luteinizing hormone; organisms causing or associated with various disease states, such as streptococcus pyogenes (group A), Herpes Simplex I and II, cytomegalovirus, rubella, chlamydiae, Candida albicans, Neisseria gonorrhoeae, Hemophilus influenzae, Group B Strep, S. pneumoniae, Neisseria meningitidis, Clostridium difficile, antibodies specific for organisms e.g. Rubella specific antibody HTLV III(HIV), etc.

The analyte may be determined in various samples, including for example, body fluids, such as saliva, urine, serum; etc. swab samples; e.g., from the throat. In some cases, it may be possible to detect analyte in whole blood.

The present invention is particularly advantageous in that the sensitivity of the assay is improved by using both a coupled compound and tracer to detect analyte. In addition, it is possible to use one tracer for assays for different analytes.

These and other advantages should be apparent to those skilled in the art from the teachings herein.

Numerous modifications and variations of the present invention are possible in light of the above, and therefore, within the scope of the appended claims, the invention may be practiced otherwise than as particularly described.

**Claims**

1. A reagent kit for use in determining an analyte, comprising a supported binder for analyte, said binder being supported on the test area of a solid support, said test area of the support being formed of a material having a surface area for supporting the supported binder, and a tracer, wherein the supported binder is supported in a concentration whereby tracer is visible on the support under assay conditions, characterised in that the kit further comprises a coupled compound comprised of a binder for the analyte and a ligand and the tracer comprises a binder for the ligand of the coupled compound labelled with a visible particulate label which is a liposome containing a detectable marker.

2. The reagent kit of Claim 1 wherein the solid support has first, second, third and fourth layers superimposed one over the other, said first layer including the test area having the supported binder supported thereon, said second layer comprising a porous material having a pore size less than the pore size of the first layer to control flow of assay reagents through the solid support, the third layer being a porous spacer layer, said fourth layer comprising an absorbent material having an absorbent capacity sufficient to absorb liquids applied to the test substrate and to provide flow through the substrate, whereby the assay for the analyte is a flow-through assay.

3. The kit of Claim 1 or 2, wherein at least the test area is formed of nitrocellulose.

4. The kit of Claim 1, 2 or 3 wherein the binder of the coupled compound is an antibody and the binder of the tracer is an antibody.

5. The kit of Claim 1, 2, 3 or 4 wherein the ligand of the coupled compound is biotin.

6. The kit of Claim 1, 2, 3 or 4 wherein the supported binder is supported on the solid support in a concentration of at least 1 ug/cm$^2$.

7. The kit of Claims 1, 2, 3, 4, 5 or 6 wherein the second layer is a nonfibrous material which provides unidirectional flow.

8. A process for assay for an analyte, comprising:
forming a complex of (i) a supported binder said supported binder being a binder for the analyte and being supported on test area of a solid support; (ii) analyte; (iii) a coupled compound comprising a binder for the analyte and a ligand; and (iv) a tracer comprised of a binder for the ligand of the coupled compound labelled with a visible particulate label which is a liposome containing a detectable marker, said test area being formed of a material having a surface area for supporting the supported binder and the supported binder being supported in a concentration whereby tracer is visible on the support under assay conditions, said analyte being determined by the visibility of the tracer in the test area.

**Patentansprüche**

1. Reagenzsatz für die Verwendung bei der Bestimmung einer zu bestimmenden Substanz, mit einem abgestützten Bindemittel für die zu bestimmende Substanz, wobei das Bindemittel auf der Prüffläche eines festen Trägers abgestützt ist und die Prüffläche des Trägers von einem Material gebildet ist, welches einen Oberflächenbereich zum Abstützen des abgestützten Bindemittels aufweist, und mit einem Markierungsmittel, worin das abgestützte Bindemittel in einer Konzentration abgestützt ist, bei welcher das Markierungsmittel auf dem Träger unter Versuchsbedingungen sichtbar ist, dadurch gekennzeichnet, daß der Reagenzsatz weiters eine aus einem Bindemittel für die zu analysierende Substanz und einem Liganden bestehende Kupplungsverbindung aufweist und das Markierungsmittel ein Bindemittel für den Liganden der Kupplungsverbindung enthält, welche mit einer sichtbaren teilchenförmigen Etikette etikettiert ist, die ein einen erkennbaren Marker enthaltendes Liposom darstellt.

2. Reagenzsatz nach Anspruch 1, worin der feste Träger eine erste Schicht, eine zweite Schicht, eine dritte Schicht und eine vierte Schicht aufweist und diese Schichten eine über der anderen angeordnet sind, wobei die erste Schicht die Prüffläche mit darauf abgestütztem abgestützten Bindemittel aufweist, die zweite Schicht ein poröses Material mit geringerer Porengroße als die erste Schicht aufweist, um den Durchfluß von Prüfreagenzien durch den festen Träger zu steuern, die dritte Schicht eine poröse Distanzschicht ist und die vierte Schicht ein absorbierendes Material aufweist, dessen Absorptionsfähigkeit ausreicht, die auf das Prüfsubstrat aufgebrachten Flüssigkeiten zu absorbieren und für einen Durchfluß durch das Substrat zu sorgen, wobei die Prüfung auf die zu bestimmende Substanz eine Durchflußprüfung ist.

3. Reagenzsatz nach Anspruch 1 oder 2, worin zumindest die Prüffläche von Nitrocellulose gebildet ist.

4. Reagenzsatz nach Anspruch 1, 2 oder 3, worin das Bindemittel für die Kupplungsverbindung ein Antikörper ist und das Bindemittel für das Markierungsmittel ein Antikörper ist.

5. Reagenzsatz nach Anspruch 1, 2, 3 oder 4, worin der Ligand der Kupplungsverbindung Biotin ist.

6. Reagenzsatz nach Anspruch 1, 2, 3 oder 4, worin das abgestützte Bindemittel auf dem festen Träger in einer Konzentration von zumindest 1 μg/cm$^2$ abgestützt ist.

7. Reagenzsatz nach den Ansprüchen 1, 2, 3, 4, 5 oder 6, worin die zweite Schicht ein nichtfasriges Material ist, welches für den Durchfluß in einer einzigen Richtung sorgt.

8. Verfahren zur Prüfung einer zu bestimmenden Substanz, bei welchem

ein Komplex aus (i) einem abgestützten Bindemittel, welches abgestützte Bindemittel ein Bindemittel für die zu bestimmende Substanz ist und auf der Prüffläche eines festen Trägers abgestützt ist, (ii) einer zu analysierenden Substanz, (iii) einer Kupplungsverbindung, welche ein Bindemittel für die zu analysierende Substanz und einen Liganden enthält, und (iv) einem Markierungsmittel, welches ein Bindemittel für den Liganden der Kupplungsverbindung enthält, welche mit einer sichtbaren teilchenförmigen Etikette etikettiert ist, die ein einen erkennbaren Marker enthaltendes Liposom darstellt, gebildet wird, wobei die Prüffläche von einem Material gebildet ist, welches einen Oberflächenbereich zum Abstützen des abgestützten Bindemittels aufweist, und wobei das abgestützte Bindemittel in einer Konzentration abgestützt ist, bei welcher das Markierungsmittel auf dem Träger unter Versuchsbedingungen sichtbar ist und wobei die zu bestimmende Substanz durch die Sichtbarkeit des Markierungsmittels in der Prüffläche bestimmt wird.

**Revendications**

1. Trousse de réactif pour utilisation dans la détermination d'un analyte comprenant un liant porté pour l'analyte, ledit liant étant porté sur la zone de test d'un support solide, ladite zone de test du support étant formée d'une matière ayant une zone de surface pour supporter le liant porté et un traceur, dans laquelle le liant porté est supporté en une concentration telle que le traceur soit visible sur le support dans les conditions de l'essai, caractérisée en ce que la trousse comprend en outre un composé couplé comprenant un liant pour l'analyte et un ligand et le traceur comprend un liant pour le ligand du composé couplé marqué avec une marque particulaire visible qui est un liposome contenant un marqueur détectable.

2. Trousse de réactif de la Revendication 1, dans laquelle le support solide a une première, deuxième, troisième et quatrième couches, les couches étant superposées l'une sur l'autre, ladite première couche comprenant la zone de test et supportant le liant porté, ladite deuxième couche comprenant une matière poreuse ayant un diamètre de pores inférieur à la dimension des pores de la première couche pour contrôler le flux des réactifs de l'essai à travers le support solide, la troisième couche étant constituée d'une couche d'écartement poreuse, ladite quatrième couche comprenant une matière absorbante ayant une capacité absorbante suffisante pour absorber les liquides appliqués sur le substrat du test et pour fournir un flux à travers le substrat, ce qui fait que l'essai pour l'analyte est un essai par circulation.

3. Trousse de la Revendication 1 ou 2, dans laquelle au moins la zone de test est formée de nitrocellulose.

4. Trousse de la Revendication 1, 2 ou 3, dans laquelle le liant du composé couplé est un anticorps et le liant du traceur est un anticorps.

5. Trousse de la Revendication 1, 2 3 ou 4, dans laquelle le ligand du composé couplé est la biotine.

6. Trousse de la Revendication 1, 2 3 ou 4, dans laquelle le liant porté est porté sur le support solide à une concentration d'au moins 1 $\mu g/cm^2$.

7. Trousse de la Revendication 1, 2, 3, 4, 5 ou 6, dans laquelle la deuxième couche est une matière non-fibreuse qui fournit un flux unidirectionnel.

8. Procédé pour l'essai d'un analyte comprenant: la formation d'un complexe de (i) un liant porté, ledit liant porté étant un liant pour l'analyte et étant porté sur la zone de test d'un support solide; (ii) l'analyte; (iii) un composé couplé comprenant un liant pour l'analyte et un ligand et (iv) un traceur comprenant un liant pour le ligand du composé couplé marqué avec une marque particulaire visible qui est un liposome contenant un marqueur détectable, ladite zone de test étant formée d'une matière ayant une zone de surface pour supporter le liant porté et le liant porté étant porté en une concentration telle que le traceur soit visible sur le support dans les conditions de l'essai, ledit analyte étant déterminé par la visibilité du traceur dans la zone de test.

EP 0 302 673 B1

# FIG. 1

# FIG. 3

# FIG.2